# EUROPÄISCHE PATENTSCHRIFT

(11) **EP 0 771 792 B1**
(45) Veröffentlichungstag und Bekanntmachung des Hinweises auf die Patenterteilung: **02.06.1999**
(21) Anmeldenummer: 96117478.6
(22) Anmeldetag: 31.10.1996
(51) Int. Cl.: C07D 263/24

(54) **Verfahren zur Herstellung von (4,5)-trans-Oxazolidin-derivaten**
Process for the preparation of (4,5)-trans-oxazolidine derivatives
Procédé pour la préparation de dérivés de la (4,5)-trans-oxazolidine

(30) Priorität: 06.11.1995 CH 3131/95
(43) Veröffentlichungstag der Anmeldung: 07.05.1997
(73) Patentinhaber: F. HOFFMANN-LA ROCHE AG, 4070 Basel (CH)
(72) Erfinder: Hilpert, Hans, 4153 Reinach (CH)
(74) Vertreter: Witte, Hubert, Dr.

(56) Entgegenhaltungen:
- EP-A- 0 635 493
- J. ORG. CHEM. 1990;, Bd. 55, Seiten 2232-2234, XP002022288 HERRANZ, R. ET AL.:
- J. MED. CHEM. 1993, Bd. 36, Nr. 17, Seiten 2431-2447, XP002022289 PATEL, D., V. ET AL.:
- BULL. SOC. CHIM. FR. 1992, Bd. 129, Seiten 585-593, XP002022290 MELON, D. ET AL.:
- TETRAHEDRON LETT. 1990, Bd. 31, Nr. 29, Seiten 4175-4167, XP002022291 MATSUMOTO, T. ET AL.:
- CHEM. PHARM BULL. 1991, Bd. 39, Nr. 10, Seiten 2550-2555, XP002022292

## Beschreibung

Die vorliegende Erfindung betrifft ein neues Verfahren zur Herstellung von (4,5)-trans-Oxazolidinen aus den entsprechenden cis-Verbindungen. Insbesondere umfasst die Erfindung Verfahren zur Herstellung von (4,5)-trans-2-Oxo-oxazolidinen der Formel (I): 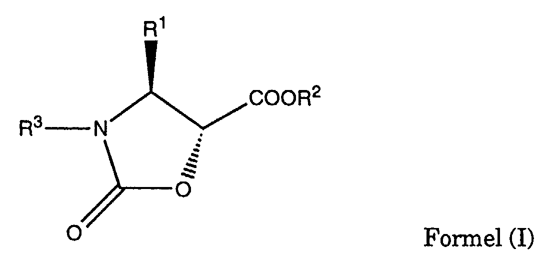 worin
- R¹: eine charakterisierende Gruppe einer α-Aminosäure,
- R²: Alkyl und
- R³: Wasserstoff, eine Amin-, Amid- oder Urethanschutzgruppe oder eine Gruppe der Formel -C(O)-R⁴ sind, wobei
- R⁴: für Alkyl oder eine oder mehrere α-Aminosäuren steht, die durch eine oder mehrere Amidbindungen miteinander verknüpft sind.

Diese Verbindungen sind wichtige Vorstufen zur Herstellung von β-Aminocarbonsäurederivaten, die Bausteine pharmakologisch wirksamer Substanzen sind, insbesondere von solchen, die sich zur Behandlung viraler Infektionen, zur Blutdrucksenkung und zur Tumorbekämpfung eignen.

Herranz et al. (J. Org. Chem. (1990) 55, 2232 - 2234) beschreiben eine Synthese von (2S,3R)-3-Amino-2-hydroxy-phenylbuttersäureestern unter Verwendung eines Trimethylsilyl-geschützten Cyanhydrins zur Herstellung von Bestatin und Amastatin.

Patel et al. (J. Med. Chem. (1993) 36, 2431 - 2447) beschreiben die Herstellung von Renininhibitoren basierend auf aktivierten Ketonen. Bei der Synthese der 2-Oxo-oxazolidin-Zwischenverbindungen ist eine aufwendige Aufarbeitung der entsprechenden Vorstufen mittels Chromatographie und Kristallisation notwendig.

Weitere Synthesewege zur Herstellung von (4,5)-trans-2-Oxo-oxazolidinen werden zum Beispiel von Melon et al. (Bull. Soc. Chim. Fr. (1992) 129, 585 - 593) beschrieben.

Es wurde nun überraschenderweise gefunden, dass das Verfahren der vorliegenden Erfindung die trans-konfigurierten Verbindungen der Formel (I) mit hoher Stereoselektivität liefert. Das erfindungsgemässe Verfahren ist dadurch gekennzeichnet, dass man ein (4,5)-cis-2-Oxo-oxazolidinon mittels einer starken Base in das entsprechende (4,5)-trans-2-Oxo-oxazolidinon überführt.

Im Detail umfasst das Verfahren die Isomerisierung von (4,5)-cis-2-Oxo-oxazolidinen der Formel (II) 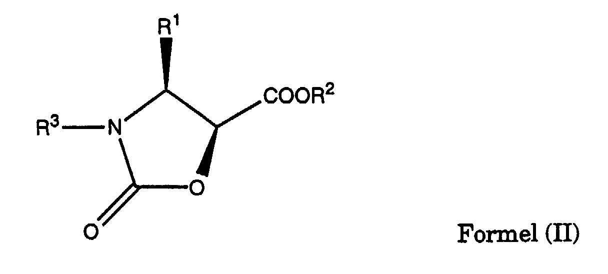 worin
- R¹: eine charakterisierende Gruppe einer α-Aminosäure, bevorzugt einer α-Aminocarbonsäure,
- R²: Alkyl und
- R³: Wasserstoff, eine Amin-, Amid- oder Urethanschutzgruppe oder eine Gruppe der Formel -C(O)-R⁴ sind, wobei R⁴ für Alkyl oder eine oder mehrere α-Aminosäuren steht, die durch eine oder mehrere Amidbindungen miteinander verknüpft sind,
mittels einer starken Base zu Verbindungen der Formel (I) 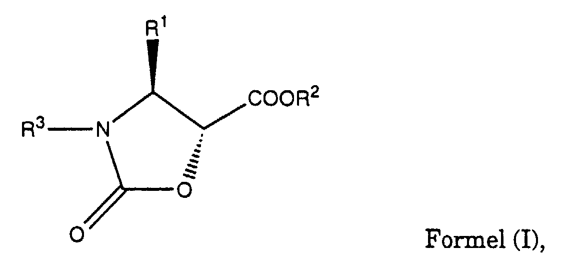 wobei R¹, R² und R³ in Formel (I) wie oben definiert sind.

Bevorzugt sind Verfahren, in denen (4,5)-cis-2-Oxo-oxazolidine der obigen Formel (II), in der R¹ eine charakterisierende Gruppe einer α-Aminocarbonsäure, R² nieder-Alkyl und R³ Wasserstoff, eine Benzyl-, Benzoyl-, Acetyl- oder eine Allylgruppe sind, isomerisiert werden.

Besonders bevorzugt sind Verfahren, in denen (4,5)-cis-2-Oxo-oxazolidine der obigen Formel (II), worin R¹ eine charakterisierende Gruppe einer natürlichen oder synthetischen α-Aminocarbonsäure, zum Beispiel Benzyl, Cyclohexylmethyl oder Phenyl ist, R² nieder-Alkyl und R³ Wasserstoff sind, isomerisiert werden.

Das erfindungsgemässe Verfahren eignet sich zum Beispiel zur Herstellung von (4S,5R)-4-Benzyl-2-oxo-oxazolidin-5-carbonsäuremethylester und (4S,5R)-4-Cyclohexylmethyl-2-oxo-oxazolidin-5-carbonsäuremethylester.

Die Reaktion wird zweckmässigerweise bei einer Temperatur zwischen -20°C und +80°C, bevorzugt zwischen +20°C und + 45°C durchgeführt.

Die in dem erfindungsgemässen Verfahren benutzten Basen sind in erster Linie starke Basen wie Alkali- oder Erdalkalialkoholate, d. h. Alkoholate, deren Kohlenwasserstoffkette Alkylreste wie für R² genannt umfasst, Lithium-, Natrium- oder Kaliumamid, Alkyllithiumverbindungen oder Alkylmagnesiumhalogenide. Bevorzugt sind Natriummethylat oder Kalium-tert.butylat.

Die Herstellung der entsprechenden cis-2-Oxo-oxazolidine bzw. entsprechender cis/trans-2-Oxo-oxazolidingemische, die als Ausgangsprodukte verwendet werden, ist dem Fachmann aus dem Stand der Technik bekannt und zum Beispiel in der Europäischen Patentanmeldung EP 0635493 beschrieben. Danach können cis-2-Oxo-oxazolidine beispielsweise durch Umsetzung eines α-Hydroxy-β-aminosäureesters mit einem entsprechenden Carbonylierungsmittels hergestellt werden. Cis-2-Oxo-oxazolidine werden zum Beispiel in der Synthese von HIV-Proteaseinhibitoren eingesetzt.

Das Verfahren wird in dem Fachmann geläufigen, inerten Lösungsmitteln durchgeführt. Der Ausdruck "inertes Lösungsmittel" bezieht sich auf ein Lösungsmittel, das unter den beschriebenen Reaktionsbedingungen inert ist. Zum Beispiel können Lösungsmittel wie Toluol, Tetrahydrofuran (THF) oder den Resten R² entsprechende Alkohole, etc. eingesetzt werden.

Im Rahmen der vorliegenden Erfindung bezeichnet der Ausdruck "eine charakterisierende Gruppe einer α-Aminosäure" die Gruppe R¹ in einer natürlichen oder synthetischen α-Aminocarbonsäure H₂NCH(R¹)COOH. Die in natürlichen α-Aminocarbonsäuren enthaltene Gruppe R¹ ist zum Beispiel Methyl (in Alanin), Isopropyl (Valin), sec.Butyl (Leucin), Methylthioäthyl (Methionin), Benzyl (Phenylalanin), 3-Indolylmethyl (Tryptophan), Hydroxymethyl (Serin), 1-Hydroxyäthyl (Threonin), Mercaptomethyl (Cystein), p-Hydroxybenzyl (Tyrosin), Carbamoylmethyl (Asparagin) Carbamoyläthyl (Glutamin), 4-Aminobutyl (Lysin), 3-Guanidinopropyl (Arginin) und 5-Imidazolylmethyl (Histidin). Als Beispiel für den Rest R¹ in einer synthetischen α-Amino- bzw. Aminocarbonsäure kann Cyclohexylmethyl und Phenyl genannt werden. Damit umfasst R¹ Alkyl-, bevorzugt nieder-Alkyl-, aber auch Aryl-, Alkoxy- und Aralkylgruppen.

Der Ausdruck "Alkyl" allein oder in Kombination bezieht sich auf einen zyklischen, verzweigten oder geradkettigen monovalenten Kohlenwasserstoffrest von einem bis vierundzwanzig, bevorzugt von einem bis zwölf Kohlenstoffatomen. Der Ausdruck "nieder-Alkyl" betrifft geradkettige oder verzweigte gesättigte Alkylreste mit 1 bis 8, vorzugsweise 1 - 4 Kohlenstoffatomen, wie Methyl, Aethyl, n-Propyl, Isopropyl, n-Butyl- Isobutyl, sec.Butyl, tert.Butyl, Pentyl, Hexyl, Heptyl, Octyl und dergleichen. Der Ausdruck "Alkoxy" allein oder in Kombination hat die Bedeutung einer Alkyläthergruppe bzw. nieder-Alkyläthergruppe, in der der Ausdruck Alkyl bzw. nieder-Alkyl die obengenannte Bedeutung besitzt, beispielsweise Methoxy, Ethoxy, n-Propoxy, Isopropoxy, n-Butoxy, Isobutoxy, sec.Butoxy, tert.Butoxy etc.

Alkyl-, nieder-Alkyl- und Alkoxygruppen können gegebenenfalls einen oder mehrere Substituenten ausgewählt aus Alkyl, Alkoxy, nieder-Alkyl, Halogen, Hydroxy, Amino, Nitro, Thio und dergleichen tragen.

Der Ausdruck "Aryl" allein oder in Kombination bedeutet eine Phenyl oder Naphthyl-Gruppe, die gegebenenfalls einen oder mehrere Substituenten ausgewählt aus Alkyl, Alkoxy, Halogen, Hydroxy, Amino, Nitro, Thio und dergleichen trägt, wie zum Beispiel Phenyl, p-Toluol, 4-Methoxyphenyl, 4-tert.Butoxyphenyl, 4-Fluorophenyl, 4-Chlorophenyl, 4-Hydroxyphenyl, 1-Naphthyl, 2-Naphthyl, etc. Der Ausdruck "Aralkyl", allein oder in Kombination bedeutet eine wie oben definierte Alkylgruppe, in der ein Wasserstoffatom durch eine Arylgruppe wie oben definiert ersetzt ist. Beispiele sind Benzyl, 2-Phenylethyl und dergleichen.

Amin-, Amid- oder Urethanschutzgruppen umfassen Gruppen wie zum Beispiel Benzyl, Benzoyl, Allyl, Acetyl oder tert.Butoxycarbonyl.

Der Ausdruck "Halogen" steht für Fluor, Chlor, Brom oder Jod.

Falls erforderlich, können Isolation und Reinigung der nach dem erfindungsgemässen Verfahren erhaltenen Verbindungen durch jede geeignete Trennungs- oder Reinigungsmethode bewerkstelligt werden, zum Beispiel durch Filtration, Extraktion, Kristallisation, Säulenchromatographie, präparative HPLC, Dünnschichtchromatographie oder Kombinationen dieser oder anderer aus dem Stand der Technik bekannter Verfahren.

Die mittels des obengenannten Verfahrens hergestellten Verbindungen können zur Synthese von pharmazeutischen Verbindungen genutzt werden, zum Beispiel bei der Synthese von Taxol. Magnus und Pye (J. Chem. Soc., Chem. Commun., (1995) 1933 - 1934) beschreiben einen Reaktionsweg zur Herstellung von Taxolderivaten. Hier kann die Taxolseitenkette an Ringsystem A durch einfache Veresterung mit den erfindungsgemässen Verbindungen eingeführt werden, wobei in einem Reaktionsschritt die korrekte Stereochemie erhalten wird.

Die folgenden Beispiele illustrieren die Erfindung.

### BEISPIEL 1

### Herstellung von (4S,5R)-4-Benzyl-2-oxo-oxazolidin-5-carbonsäuremethylester

Eine Lösung von 70.0 g einer 92:8-Gemisches der (4S,5S)- und (4S,5R)-Isomeren des 4-Benzyl-2-oxo-oxazolidin-5-carbonsäuremethylesters und 7.0 g Kalium-tert.butylat in 700 ml THF wurde vier Stunden bei 20°C und 2 Stunden bei 45°C gerührt, die Lösung zweimal mit halbgesättigter Kochsalzlösung gewaschen, über MgSO₄ getrocknet, filtriert und das Filtrat zu 68 g (97 %) 95 %-igem (4S,5R)-4-Benzyl-2-oxo-oxazolidin-5-carbonsäuremethylester eingedampft (DC (Si0₂, Aethylacetat): R_{f} = 0.5).

### BEISPIEL 2

### Herstellung von (4S,5R)-4-Cyclohexylmethyl-2-oxo-oxazolidin-5-carbonsäuremethylester

Zur Herstellung der obengenannten Verbindung wird (4S,5S)-4-Cyclohexylmethyl-2-oxo-oxazolidin-5-carbonsäuremethylester, erhältlich durch Hydrierung von (4S,5R)-4-Benzyl-2-oxo-oxazolidin-5-carbonsäuremethylester, unter den in Beispiel 1 angegebenen Reaktionsbedingungen umgesetzt. Man erhält (4S,5R)-4-Cyclohexylmethyl-2-oxo-oxazolidin-5-carbonsäuremethylester mit einer Ausbeute von ca. 95 %.

## Patentansprüche

1. Verfahren zur Herstellung von (4,5)-trans-2-Oxo-oxazolidinen, dadurch gekennzeichnet, dass (4,5)-cis-2-Oxo-oxazolidine der Formel (II) 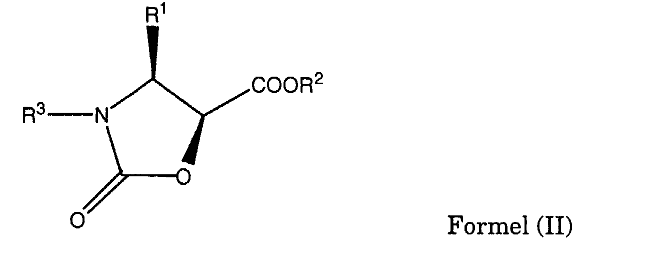 worin
R¹ eine charakterisierende Gruppe einer α-Aminosäure,
R² Alkyl und
R³ Wasserstoff, eine Amin-, Amid- oder Urethanschutzgruppe oder eine Gruppe der Formel -C(O)-R⁴ sind, wobei R⁴ für Alkyl oder eine oder mehrere α-Aminosäuren steht, die durch eine oder mehrere Amidbindungen miteinander verknüpft sind,
mittels eines Alkali- oder Erdalkalialkoholats, eines Lithium-, Natrium- oder Kaliumamids, einer Alkyllithiumverbindung oder eines Alkylmagnesiumhalodgenids zu Verbindungen der Formel (I) 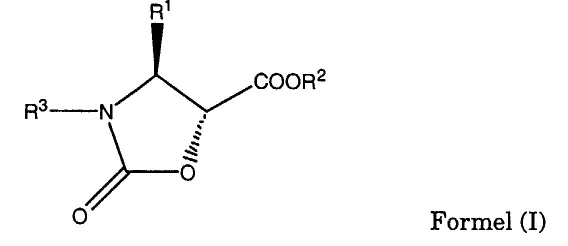 isomerisiert werden,
wobei R¹, R² und R³ in Formel (I) wie oben definiert sind.

2. Verfahren gemäss Anspruch 1, dadurch gekennzeichnet, dass R¹ eine charakterisierende Gruppe einer α-Aminocarbonsäure, R² nieder-Alkyl und R³ Wasserstoff, Acetyl, eine Benzyl-, Benzoyl- oder eine Allylgruppe ist.

3. Verfahren gemäss Anspruch 2 dadurch gekennzeichnet, dass R¹ eine charakterisierende Gruppe einer natürlichen oder synthetischen α-Aminocarbonsäure ist, R² nieder-Alkyl und R³ Wasserstoff ist.

4. Verfahren gemäss Anspruch 3, dadurch gekennzeichnet, dass R¹ Phenyl, Cyclohexylmethyl oder Benzyl ist.

5. Verfahren gemäss einem der Ansprüche 1-4, dadurch gekennzeichnet, dass als Isomerisierungsmittel Natriummethylat oder Kalium-tert.butylat verwendet wird.

6. Verfahren gemäss einem der Ansprüche 1-5, dadurch gekennzeichnet, dass es bei einer Temperatur von -20°C bis +80°C durchgeführt wird.

7. Verfahren gemäss einem der Ansprüche 1-6, dadurch gekennzeichnet, dass es bei einer Temperatur von +20°C bis +45°C durchgeführt wird.

8. Verfahren gemäss einem der Ansprüche 1-7, dadurch gekennzeichnet, dass als Lösungsmittel Toluol, Tetrahydrofuran oder ein Alkohol verwendet wird.

9. Verfahren gemäss einem der Ansprüche 1-8, dadurch gekennzeichnet, dass als Ausgangsprodukt ein cis/trans-2-Oxo-oxazolidingemisch eingesetzt wird.

## Claims

1. A process for the manufacture of (4,5)-trans-2-oxo-oxazolidines, characterized by isomerizing (4,5)-cis-2-oxo-oxazolidines of formula (II) 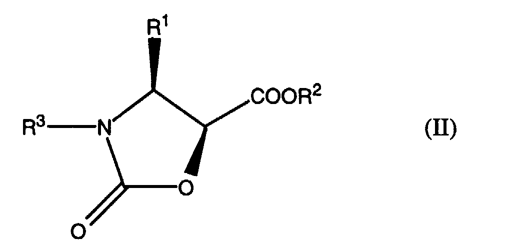 wherein
R¹ is a characterizing group of an α-amino acid,
R² is alkyl and
R³ is hydrogen, an amine, amide or urethane protecting group or a group of the formula -C(O)-R⁴ in which R⁴ stands for alkyl or one or more α-amino acids which are linked with one another by one or more amide bonds,
by means of an alkali metal or alkaline earth metal alcoholate, a lithium, sodium or potassium amide, an alkyllithium compound or an alkylmagnesium halide to give compounds of formula (I) 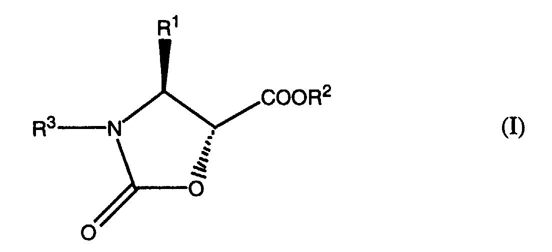 in which R¹, R² and R³ in formula (I) are as defined above.

2. A process according to claim 1, characterized in that R¹ is a characterizing group of an α-aminocarboxylic acid, R² is lower-alkyl and R³ is hydrogen, acetyl, a benzyl, benzoyl or an allyl group.

3. A process according to claim 2, characterized in that R¹ is a characterizing group of a natural or synthetic α-aminocarboxylic acid, R² is lower-alkyl and R³ is hydrogen.

4. A process according to claim 3, characterized in that R¹ is phenyl, cyclohexylmethyl or benzyl.

5. A process according to any one of claims 1-4, characterized in that sodium methylate or potassium tert.butylate is used as the strong base.

6. A process according to any one of claims 1-5, characterized in that it is carried out at a temperature of -20°C to +80°C.

7. A process according to any one of claims 1-6, characterized in that it is carried out at a temperature of +20°C to +45°C.

8. A process according to any one of claims 1-7, characterized in that toluene, tetrahydrofuran or an alcohol is used as the solvent.

9. A process according to any one of claims 1-8, characterized in that a cis/trans-2-oxo-oxazolidine mixture is used as the starting product.

## Revendications

1. Procédé pour la préparation de (4,5)-trans-2-oxo-oxazolidines, caractérisé en ce que l'on isomérise des (4,5)-cis-2-oxo-oxazolidines de formule II 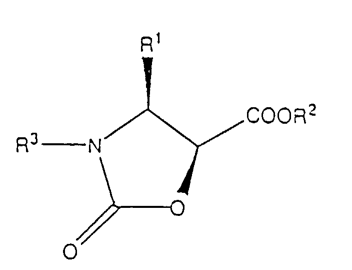 dans laquelle
R¹ représente un groupement caractérisant d'un α-aminoacide,
R² représente un groupe alkyle et
R³ représente l'hydrogène, un groupe protecteur d'une fonction amine, amide ou uréthanne, ou un groupe de formule -C (0)-R⁴ dans laquelle R⁴ représente un groupe alkyle ou un ou plusieurs α-aminoacides reliés entre eux par une ou plusieurs liaisons amides,
à l'aide d'un alcoolate alcalin ou alcalino-terreux, d'un amidure de lithium, de sodium ou de potassium, d'un dérivé d'alkyllithium ou d'un halogénure d'alkylmagnésium, en composés de formule I 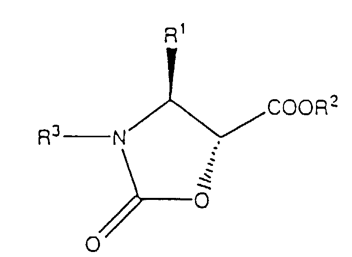 dans laquelle R¹, R² et R³ ont les significations indiquées ci-dessus.

2. Procédé selon la revendication 1, caractérisé en ce que R¹ représente un groupe caractérisant d'un acide α-aminocarboxylique, R² représente un groupe alkyle inférieur et R³ l'hydrogène, un groupe acétyle, benzyle, benzoyle ou allyle.

3. Procédé selon la revendication 2, caractérisé en ce que R¹ représente un groupe caractérisant d'un acide α-aminocarboxylique naturel ou synthétique, R² représente un groupe alkyle inférieur et R³ l'hydrogène.

4. Procédé selon la revendication 3, caractérisé en ce que R¹ représente un groupe phényle, cyclohexylméthyle ou benzyle.

5. Procédé selon l'une des revendications 1 à 4, caractérisé en ce que l'on utilise en tant qu'agent isomérisant le méthylate de sodium ou le tert-butylate de potassium.

6. Procédé selon l'une des revendications 1 à 5, caractérisé en ce que l'on opère à une température allant de -20 à +80°C.

7. Procédé selon l'une des revendications 1 à 6, caractérisé en ce que l'on opère à une température de +20 à +45°C.

8. Procédé selon l'une des revendications 1 à 7, caractérisé en ce que l'on utilise le toluène, le tétrahydrofuranne ou un alcool en tant que solvant.

9. Procédé selon l'une des revendications 1 à 8, caractérisé en ce que le produit mis en oeuvre au départ est un mélange de cis/trans-2-oxo-oxazolidines.
